# FASCICULE DE BREVET EUROPEEN

(11) **EP 0 357 742 B1**
(45) Date de publication et mention de la délivrance du brevet: **09.06.1993**
(21) Numéro de dépôt: 89903169.4
(22) Date de dépôt: 03.03.1989
(51) Int. Cl.: C07C 69/74, C07C 69/743, C07C 69/747, C07C 35/32, C07C 35/52, A01N 53/00, A61K 31/215

(54) **NOUVEAUX ESTERS PYRETHRINOIDES PORTANT UN NOYAU INDANYLE, LEUR PROCEDE DE PREPARATION ET LEUR APPLICATION COMME PESTICIDES**
INDANYLRING ENTHALTENDE PYRETHINESTER, DEREN HERSTELLUNG UND DEREN ANWENDUNG ALS PESTIZIDE
NEW PYRETHRINOID ESTERS CARRYING AN INDANYL RING, PREPARATION METHOD AND APPLICATION AS PESTICIDES

(30) Priorité: 03.03.1988 FR 8802694
(43) Date de publication de la demande: 14.03.1990
(73) Titulaire: ROUSSEL-UCLAF, 75007 Paris (FR)
(72) Inventeur: BABIN, Didier, F-78180 Montigny (FR); DEMOUTE, Jean-Pierre, F-93360 Neuilly-Plaisance (FR); TESSIER, Jean, F-94300 Vincennes (FR)
(74) Mandataire: Tonnellier, Marie-José
(86) Numéro de dépôt international: FR8900087
(87) Numéro de publication internationale: WO8908096

(56) Documents cités:
- EP-A- 0 003 336
- EP-A- 0 063 418
- EP-A- 0 215 701
- US-A- 3 647 857
- US-A- 4 263 319

## Description

La présente invention concerne de nouveaux esters pyréthrinoïdes portant un noyau indanyle, leur procédé de préparation et leur application comme pesticides.

La demande européenne publiée sous le numéro EP-A-215701 décrit les composés de formule : 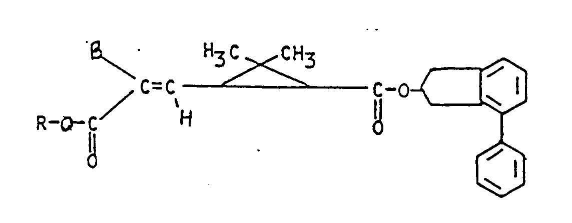
dans laquelle B représente un atome d'hydrogène ou d'halogène
et R représente un radical alcoyle, linéaire, ramifié ou cyclique, saturé ou insaturé, renfermant de 1 à 8 atomes de carbone éventuellement substitué par un ou plusieurs groupements fonctionnels, identiques ou différents, le document décrit également la préparation de ces composés, leur application à la lutte contre les parasites et les compositions les renfermant.

L'invention a pour objet sous toutes les formes isomères possibles, ainsi que leurs mélanges, les composés de formule (I) : 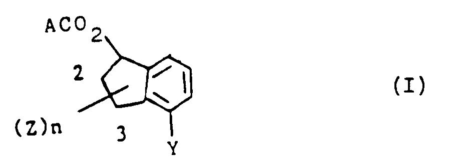
dans laquelle A représente le radical : 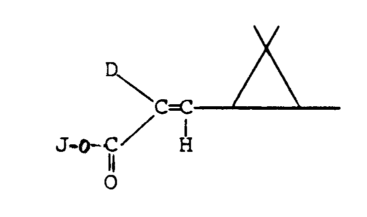
dans lequel D représente un atome d'hydrogène ou de fluor, et J représente un radical alkyle linéaire, ramifié ou cyclique, saturé ou insaturé, renfermant jusqu'à 4 atomes de carbone, éventuellement substitué par un ou plusieurs atomes d'halogène,
- Z en position 2 ou 3 représente un atome d'hydrogène, un atome de fluor, un radical alkyle linéaire, ramifié ou cyclique, saturé ou insaturé renfermant jusqu'à 8 atomes de carbone, n représentant le nombre 1, 2 ou 3 ;
- Y représente le radical -C≡N, CH₂CH=CH₂, CH₂-C≡CH ou -CH₂-C≡N.

Lorsque Z représente un radical alkyle saturé linéaire ou ramifié, il s'agit de préférence d'un radical méthyle, éthyle, n-propyle, isopropyle, n-butyle, sec-butyle, isobutyle, n-pentyle, n-hexyle, tert-butyle ou tert-pentyle ou néo-pentyle.

Lorsque Z représente un radical alkyle insaturé, il s'agit de préférence d'un radical éthylénique comme, par exemple, d'un radical vinyle, allyle, 1,1-diméthylallyle, 2-butényle ou d'un radical acétylénique comme, par exemple, le radical éthynyle ou propynyle.

Lorsque Z, représente un radical alkyle cyclique, il s'agit de préférence d'un radical cyclopropyle, cyclobutyle, cyclopentyle ou cyclohexyle.

Lorsque J représente un radical alkyle substitué par un ou plusieurs atomes d'halogène , on entend de préférence par alkyle le radical méthyle, éthyle, propyle, isopropyle, n-butyle, isobutyle ou tert-butyle On peut citer comme valeurs préférées de J, les radicaux : 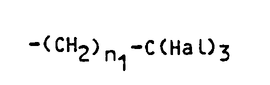
dans lequel n₁ est un entier de 1 à 3 et Hal un atome d'halogène, par exemple le radical -CH₂-CCl₃, -CH₂-CF₃, -CH₂-CH₂-CCl₃ ou -CH₂-CH₂-CF₃; 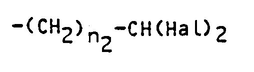
dans lequel Hal est défini comme ci-dessus et n₂ est un nombre de 0 à 3, par exemple le radical -CH₂-CHCl₂, -CH₂-CHF₂ ou -CHF₂; 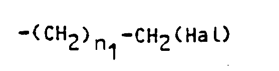
dans lequel n₁ et Hal sont définis comme ci-dessus par exemple le radical -CH₂-CH₂Cl ou -CH₂-CH₂F, -C [C(Hal)₃]₃ dans lequel Hal est défini comme ci-dessus, par exemple le radical -C (CF₃)₃ ou 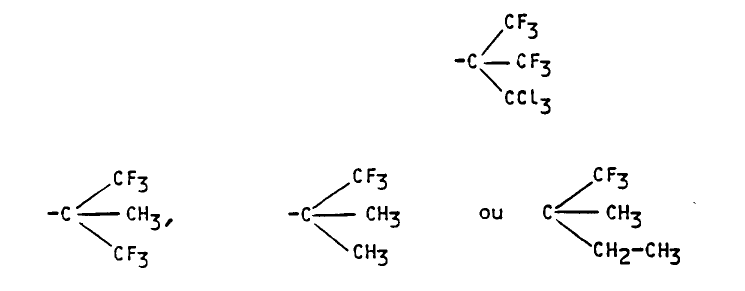 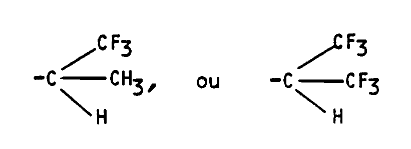
Parmi les composés préférés de l'invention, on peut naturellement citer les composés dont la préparation est donnée ci-après dans la partie expérimentale et notamment les composés des exemples 1, 2, 3, 4, 5, 25, 26, 45.

Les composés de formule (I) présentent d'intéressantes propriétés qui permettent leur utilisation dans la lutte contre les parasites ; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites des animaux à sang chaud.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 1 g et 300 g de matière active à l'hectare.

Les produits de formule (I) peuvent également être utilisés pour lutter contre les insectes dans les locaux, pour lutter notamment contre les mouches, les moustiques et les blattes.

Parmi les produits préférés de l'invention, on peut citer ceux des exemples 1, 2, 3, 4, 5, 25, 26 et 45.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii),poudre de tige de pyrèthre, poudre de feuille de cèdre, poudre de bois (telle que la sciure de pin) amidon et poudre de coque de noix de coco. La dose de matière active peut alors être, par exemple, de 0,03 à 1 % en poids.

Dans le cas où l'on utlise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins un des produits de formule (I) définie ci-dessus.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire, contenant de 1 à 500 g/l de principe actif. On peut également employer des poudres pour poudrage foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Pour exalter l'activité biologique des produits de l'invention, on peut les additionner à des synergistes classiques utilisé en pareil cas tel le 1-(2,5,8-trioxadodécyl) 2-propyl 4,5-méthylènedioxy benzène (ou butoxyde de pipéronyle) ou la N-(2-éthyl heptyl) bicyclo[2,2-1] hept-5-èn-2,3-dicarboximide, ou le pipéronyl bis [2-(2'-n-butoxy éthoxy) éthyl] acétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des anti-oxydants.

L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcool-alpha-cyano 3-phénoxy benzyliques d'acides 2,2-diméthyl 3-(2,2-dibromovinyl) cyclopropane carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido-méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahalo éthyl)cyclopropane carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présente notamment l'intérêt soit de permettre de combattre, par la polyvalence de leur action, une gamme de parasites, plus étendue, soit de manifester, dans certains cas, un effet de synergie.

L'invention a pour objet les compositions pesticides définies précédemment, caractérisées en ce qu'elles renferment en outre un synergiste des pyréthrinoïdes.

Comme synergistes classiques utilisés en pareil cas, on peut citer le 1-(2,5,8-trioxadodécyl) 2-propyl 4,5-méthylène dioxy benzène (ou butoxyde de pipéronyle) ou le N-(2-éthyl heptyl) bicyclo [2,2-1] hept-5-èn-2,3-dicarboximide, ou le pipéronal bis[2-(2'-n-butoxy éthoxy) éthyl] acétal (ou tropital).

L'invention a également pour objet un procédé de préparation des composés de formule (I), caractérisé en ce que l'on soumet un acide de formule (II) : 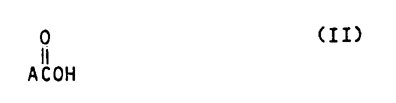
dans laquelle A est défini comme précédemment ou un dérivé fonctionnel de cet acide à l'action d'un alcool de formule (III) : 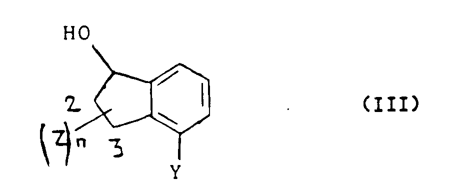
dans laquelle Y, Z et n sont définis comme précédemment pour obtenir le composé de formule (I) correspondant.

Les composés de formule (III) sous forme racémique sont des composés connus d'une façon générale, décrits ou envisagés dans Agr. Biol. Chem. 1978, 42, 1365.

Les composés de formule (III) sous forme dédoublée peuvent être préparés par hydrolyse enzymatique des esters correspondants. Pour réaliser l'hydrolyse enzymatique, on peut utiliser une lipase par exemple la lipase de pancréas de porc, comme il sera indiqué ci-après dans la partie expérimentale.

La préparation de certains composés de formule (III) sous forme dédoublée ou sous forme racémique est donnée ci-après dans la partie expérimentale.

Les exemples suivants illustrent l'invention, sans toutefois la limiter.

### Exemple 1 : [1R-[1alpha(RS*),3alpha(Z)]] 3-[3-(1,1-diméthyléthoxy) 3-oxo 1-propényl] 2,2-diméthyl cyclopropanecarboxylate de 4-(2-propényl) 1-indanyle.

On mélange 0,58 g d'acide [1Rcis (Z)] 2,2-diméthyl (1,1-diméthyléthoxy) 3-oxo 1-propényl cyclopropanecaboxylique 6 ml de chlorure de méthylène, 0,42 g de (RS) 4-(2-propényl) 1-indanol, 50 mg de 4-diméthylamino pyridine et refroidit à 0¤C. On ajoute goutte à goutte 0,51 g de dicyclohexylcarbodiimide dans 1,5 ml de chlorure de méthylène et agite durant 17 heures à température ambiante. On essore l'urée formée et concentre à sec le filtrat sous pression réduite. On chromatographie le résidu sur silice, élue par un mélange hexane-éther isopropylique (9-1) et sépare 0,43 g de produit impur et 0,46 g de produit attendu pur. Après chromatographie sur silice, on purifie la fraction impure, en éluant par un mélange hexane-éther isopropylique (9,5-0,5) et récupère encore 0,34 g de produit pur.
[alpha]_{D} = +53,5¤ ±2,5 c = 0,4 % CHCl₃
L'alcool utilisé au départ est préparé selon le procédé décrit dans Agr. Biol. Chem. 1978 42 1365.

### Exemple 2 : [1R-[1alpha(RS*),3alpha(E)]] 3-(2-fluoro 3-éthoxy 3-oxo 1-propényl) 2,2-diméthyl cyclopropanecarboxylate de 4-(2-propényl) 1-indanyle.

[alpha]_{D} = +20,7 ±2¤ c = 0,4 % CHCl₃

### Exemple 3 : [1R-[1alpha,3alpha(E)]] 3-[3-(1,1-diméthyléthoxy) 2-fluoro 3-oxo 1-propènyl] 2,2-diméthyl cyclopropanecarboxylate de 4-(2-propényl) 1-indanyle.

[alpha]_{D} = +31¤ ± 1¤ c=1% CHCl₃

### Exemple 4 : [1R-[1alpha(R*),3alpha(E)]] 3-(3-éthoxy 2-fluoro 3-oxo 1-propényl) 2,2-diméthyl cyclopropanecarboxylate de 4-(2-propényl) 1-indanyle

[alpha]_{D} = +98¤ ± 2¤ 1% CHCl₃
Alcool : (voir préparation 22).

### Exemple 5 : [1R-[1alpha(R*) 3alpha(E)]] 3-[3-(1,1-diméthyléthoxy) 2-fluoro 3-oxo 1-propényl 2,2-diméthyl cyclopropanecarboxylate de 4-(2-propényl) 1-indanyle.

[alpha]_{D} +104,5¤ ± 2¤ c=0,85% CHCl₃

### Exemple 6 : [1R-[1alpha(S*) 3alpha(E)]] 3-[3-(3-éthoxy 2-fluoro 3-oxo 1-propényl) 2,2-diméthyl cyclopropanecarboxylate de 4-(2-propényl) 1-indanyle.

[alpha]_{D} = -53¤ ± 1,5¤ c=1% CHCl₃

### Exemple 7 : [1R-[1alpha(S*) 3alpha(E)]] 3-[3-(1,1-diméthyléthoxy) 2-fluoro 3-oxo 1-propényl] 2,2-diméthyl cyclopropanecarboxylate de 4-(2-propényl) 1-indanyle.

[alpha]_{D} = -33¤ ± 1,5¤ c=1% CHCl₃

### Exemple 8 : [1R-[1alpha,3alpha(Z)]] 3-[3-(1,1-diméthyléthoxy) 3-oxo 1-propényl] 2,2-diméthyl cyclopropanecarboxylate de [cis(±) 2-fluoro 4-(1-propényl) 1-indanyle].

[alpha]_{D} : +62,5 ± 1,5¤ c = 1% CHCl₃
Alcool utilisé : (voir préparation 1).

### Exemple 9 : [1R-[1alpha,3alpha(E)]] 3-[3-éthoxy 2-fluoro 3-oxo 1-propényl] 2,2-diméthyl cyclopropanecarboxylate de [cis(±) 2-fluoro 4-(2-propényl) 1-indanyle].

[alpha]_{D} = +37,5 ± 1,5¤ c = 1% CHCl₃

### Exemple 10 : [1R-[1alpha,3alpha(E)]] 3-[3-éthoxy 2-fluoro 3-oxo 1-propényl) 2,2-diméthyl cyclopropanecarboxylate de [(RS,cis) 2-chloro 4-(2-propényl) 1-indanyle].

[alpha]_{D} = +30,5¤ ± 1¤ c=1% CHCl₃
Alcool utilisé : (voir préparation 2).

### Exemple 11 : [1R-[1alpha,3alpha(Z)]] 3-[3-(1,1-diméthyléthoxy 3-oxo 1-propényl] 2,2-diméthyl cyclopropanecarboxylate de [(RS,cis) 2-chloro 4-(2-propényl) 1-indanyle.

[alpha]_{D} = -53,5¤ ± 1,5¤ c=1% CHCl₃

### Exemple 12 : [1R-[1alpha,3alpha(E)]] 3-[3-éthoxy 2-fluoro 3-oxo 1-propényl] 2,2-diméthyl cyclopropanecarboxylate de (RS,trans) 2-chloro 4-(2-propényl) 1-indanyle].

[alpha]_{D} = +29¤ ± 1¤ c=1% CHCl₃

### Exemple 13 : [1R-[1alpha,3alpha(E)]] 3-[3-éthoxy 2-fluoro 3-oxo 1-propényl] 2,2-diméthyl cyclopropanecarboxylate de 2-méthyl 4-(2-propényl) 1-indanyle.

[alpha]_{D} = +26,5¤ ± 2,5¤ c=0,6% CHCl₃
Alcool utilisé : (voir préparation 3).

### Exemple 14 : [1R-[1alpha,3alpha(Z)]] 3-[3-(1,1-diméthyléthoxy) 3-oxo 1-propényl] 2,2-diméthyl cyclopropanecarboxylate de 2-méthyl 4-(2-propényl) 1-indanyle.

[alpha]_{D} = +51¤5 ± 1,5¤ c=0,7%

### Exemple 15 : [1R-[1alpha(RS*),3alpha(E)]] 3-(3-éthoxy 2-fluoro 3-oxo 1-propényl) 2,2-diméthyl cyclopropanecarboxylate de 5-(2-propényl) 1-indanyle.

[alpha]_{D} +24¤ ± 2¤ c=0,5% CHCl₃
Alcool voir préparation 4.

### Exemple 16 : [1R-[1alpha(RS*),3alpha (Z)]] 3-[3-(1,1-diméthyléthoxy) 3-oxo 1-propényl] 2,2-diméthyl cyclopropanecarboxylate de 5-(2-propényl) 1-indanyle.

[alpha]_{D} = +55¤5 ± 1,5¤ c=0,8% CHCl₃

### Exemple 17 : [1R-[1alpha(RS*),3alpha(E)]] 3-[3-(1,1-diméthyléthoxy) 2-fluoro 3-oxo 1-propényl] 2,2-diméthyl cyclopropanecarboxylate de 6-(2-propényl) 1-indanyle.

[alpha]_{D} = +37¤ ± 2,5¤ c=0,45% CHCl₃
Voir préparation 5 pour l'alcool utilisé.

### Exemple 18: [1R-[1alpha(RS*),3alpha(E)]] 3-(3-éthoxy 2-fluoro 3-oxo 1-propényl] 2,2-diméthyl cyclopropanecarboxylate de 6-(2-propényl) 1-indanyle.

[alpha]_{D} = +20¤ ± 2,5¤ c=0,45% CHCl₃

### Exemple 19: [1R-(1alpha(RS*),3alpha(E)]] 3-(3-éthoxy 2-fluoro 3-oxo 1-propényl) 2,2-diméthyl cyclopropanecarboxylate de 1-indanyle.

[alpha]_{D} = +28¤ ± 2¤
Alcool utilisé voir préparation 6.

### Exemple 20: [1R-[1alpha(RS*),3alpha(Z)]] 3-[3-(1,1-diméthyléthoxy) 3-oxo 1-propényl] 2,2-diméthyl cyclopropanecarboxylate de 1-indanyle.

[alpha]_{D} = +59,5¤ ± 2,5¤ c=0,4% CHCl₃

### Exemple 21 : [1R-[1alpha(RS*),3alpha(Z)] 3-[3-(1,1-diméthyléthoxy) 3-oxo 1-propényl] 2,2-diméthyl cyclopropanecarboxylate de 4-bromo 1-indanyle.

[alpha]_{D} = +44,5¤ ± 2¤ c=0,4% CHCl₃
Alcool utilisé voir Agr. Biol. Chem. 1978, 42, 1365.

### Exemple 22: [1R-[1alpha(RS*),3alpha(E)]] 3-(3-éthoxy 2-fluoro 3-oxo 1-propényl) 2,2-diméthyl cyclopropanecarboxylate de 4-bromo 1indanyle.

[alpha]_{D} = +22¤ ± 1¤ c=0,7% CHCl₃

### Exemple 23 : [1R-[1alpha(RS*),3alpha(E)]] 3-[3-(1,1-diméthyléthoxy) 2-fluoro 3-oxo 1-propényl] 2,2-diméthyl cyclopropanecarboxylate de 4-bromo 1-indanyle.

[alpha]_{D} = +23¤ ± 1¤ c=0,7% CHCl₃

### Exemple 24 : [1R-[1alpha(RS*),3alpha (Z)]] 3-[3-(1,1-diméthyléthoxy) 3-oxo 1-propényl] 2,2-diméthyl cyclopropanecarboxylate de 4-cyano 1-indanyle.

[alpha]_{D} = +58,5¤ ± 2¤ c=0,75% CHCl₃
Alcool voir préparation 7. Agr. Biol. Chem. (1978), 42, 1365.

### Exemple 25 : [1R-[1alpha(RS*),3alpha(E)]] 3-(3-éthoxy 2-fluoro 3-oxo 1-propényl) 2,2-diméthyl cyclopropanecarboxylate de 4-cyano 1-indanyle.

[alpha]_{D} = +25,5¤ ± 2¤ c=0,5% CHCl₃

### Exemple 26 : [1R-[1alpha(RS*),3alpha(E)]] 3-(3-(diméthyléthoxy) 2-fluoro 3-oxo 1-propényl) 2,2-diméthyl cyclopropanecarboxylate de 4-cyano 1-indanyle.

[alpha]_{D} = +33¤ ± 2,5¤ c=0,5% CHCl₃

### Exemple 27 : [1R-[1alpha(S*),3alpha(E)]] 3-[3-(1,1-diméthyléthoxy) 2-fluoro 3-oxo 1-propényl] 2,2-diméthyl cyclopropanecarboxylate de 4-cyano-1-indanyle.

[alpha]_{D} = -51¤ ± 1,5¤ c=0,75% CHCl₃
Alcool (S) : voir préparation 8 (Stade C).

### Exemple 28 : [1R-[1alpha(S*),3alpha(E)]] 3-(3-éthoxy 2-fluoro 3-oxo 1-propényl) 2,2-diméthyl cyclopropanecarboxylate de 4-cyano 1-indanyle.

[alpha]_{D} = -77¤ + 2,5¤ c=0,5%

### Exemple 29 : [1R-[1alpha(R*),3alpha(E)]] 3-(3-éthoxy 2-fluoro 3-oxo 1-propényl) 2,2-diméthyl cyclopropanecarboxylate de 4-cyano 1-indanyle.

[alpha]_{D} = +138¤ ± 2,5¤ c=0,8% CHCl₃
Alcool (R) : Voir préparation 9 (Stade B).

### Exemple 30 : [1R-[1alpha(R*),3alpha(E)]] 3-[3-(1,1-diméthyléthoxy) 2- fluoro 3-oxo 1-propényl] 2,2-diméthyl cyclopropanecarboxylate de 4-cyano 1-indanyle.

[alpha]_{D} = +135¤ ± 2,5¤ c=0,7% CHCl₃

### Exemple 31 : [1R-[1alpha(RS*),3alpha(E)]] 3-(3-éthoxy 2-fluoro 3-oxo 1-propényl) 2,2-diméthyl cyclopropanecarboxylate de 5-cyano 1-indanyle.

[alpha]_{D} = +40,5¤ ± 2,5¤ c=0,4% CHCl₃
Alcool voir préparation 8.

### Exemple 32 : [1R-[1alpha(RS*),3alpha(Z)]] 3-[3-(1,1-diméthyléthoxy) 3-oxo-1-propényl] 2,2-diméthyl cyclopropanecarboxylate de 5-cyano 1-indanyle.

[alpha]_{D} = 61,5¤ ± 1,5¤ c=1% CHCl₃

### Exemple 33 : [1R-[1alpha(RS*),3alpha(E)]] 3-(3-éthoxy 2-fluoro 3-oxo 1-propényl) 2,2-diméthyl cyclopropanecarboxylate de 6-cyano 1-indanyle.

[alpha]_{D} = +29,5¤ ± 1,5¤ c=1% CHCl₃
Alcool voir préparation 10.

### Exemple 34 : [1R-[1alpha(RS*),3alpha(E)]] 3-[3-(1,1-diméthyléthoxy 2-fluoro 3-oxo 1-propényl] 2,2-diméthyl cyclopropanecarboxylate de 6-cyano 1-indanyle.

[alpha]_{D} = +41¤ ± 1,5¤ c=1% CHCl₃

### Exemple 35 : [1R-[1alpha(RS*),3alpha(E)]] 3-[3-(1,1-diméthyléthoxy) 2-fluoro 3-oxo 1-propényl] 2,2-diméthyl cyclopropanecarboxylate de 7-cyano 1-indanyle.

[alpha]_{D} = +18¤ ± 1¤ c=1% CHCl₃
Alcool voir préparation 11.

### Exemple 36 : [1R-[1alpha(RS*),3alpha(E)]] 3-(3-éthoxy 2-fluoro 3-oxo 1-propényl) 2,2-diméthyl cyclopropanecarboxylate de 7-cyano 1-indanyle.

[alpha]_{D} = -5¤ ± 1¤ c=0,7% CHCl₃

### Exemple 37 : [1R-[1alpha(RS*),3alpha(E)]] 3-(3-éthoxy 2-fluoro 3-oxo 1-propényl) 2,2-diméthyl cyclopropanecarboxylate de 4-cyanométhyl 1-indanyle.

[alpha]_{D} = +23,5¤ ± 1,5¤ c=0,6% CHCl₃
Voir préparation 12 (alcool RS).

### Exemple 38 : [1R-[1alpha(RS*),3alpha(Z)]] 3-[3-(1,1-diméthyléthoxy) 3-oxo-1-propényl] 2,2-diméthyl cyclopropanecarboxylate de 4-cyanométhyl-1-indanyle.

[alpha]_{D} = +54,5¤ ± 1¤ c=1% CHCl₃

### Exemple 39 : [1R-[1alpha(RS*),3alpha(E)]] (3-éthoxy 2-fluoro 3-oxo 1-propényl) 2,2-diméthyl cyclopropanecarboxylate de 4-(1-cyano 2-éthoxy 2-oxo-éthyl) 1-indanyle.

### Spectre RMN CDCl₃ 60 MHz ppm :

1,16-1,46 :H des groupes méthyle géminés et en position 2 sous-groupes éthoxy
4,07-4,45 : H en position 1 d'un sous-groupe éthoxy
1,8-1,94-2,08 à 3,31 : H en position 1 et 3 du cyclopropane et en position 2
et 3 du groupe 1-indanyle ; 7,35 : aromatiques
4,74 : H en alpha du CN
6,08-6,16-6,25 : H en position 1 d'un sous-groupe éthoxy
6,16-6,33-6,5-6,6 : H éthyléniques
Alcool RS voir préparation 17

### Exemple 40 : [1R-[1alpha(RS*),3alpha(Z)]] 3-[3-(1,1-diméthyléthoxy) 3-oxo-1-propényl] 2,2-diméthyl cyclopropanecarboxylate de 4-(1-cyano 2-éthoxy-2-oxo éthyl) 1-indanyle.

### Spectre RMN CDCl₃ 60 MHz ppm :

1,25-1,31 : CH₃ gem
1,47 : tBu
1,16-1,28-1,40 : H en position 2 du groupe éthyle
4,06-4,18-4,3-4,38 : H en position 1 du groupe éthyle
1,82 à 1,95 H₁ cis du cyclopropyle
2,13 à 3,38 H₃ du cyclopropyle et les CH₂
4,74 H en alpha du CN
5,68-5,87-6,34-6,53-6,50-6,7 : H éthyléniques
7,35 : aromatiques
Voir préparation 13 (alcool RS)

### Exemple 41 : [1R-[1alpha(RS*),3alpha(E)]] 3-(3-éthoxy 2-fluoro 3-oxo 1-propényl) 2,2-diméthyl cyclopropanecarboxylate de 4-propyl 1-indanyle.

[alpha]_{D} = +18¤ ± 2¤ c=0,6% CHCl₃
Alcool utilisé voir préparation 14 .

### Exemple 42 : [1R-[1alpha(RS*),3alpha(E)]] 3-(3-diméthyléthoxy 2-fluoro 3-oxo 1-propényl) 2,2-diméthyl cyclopropanecarboxylate de 4-propyl 1-indanyle.

[alpha]_{D} = +35,5¤ c=1% CHCl₃

### Exemple 43 : [1R-[1alpha,3alpha(E)]] 3-(3-éthoxy 2-fluoro 3-oxo 1-propényl) 2,2-diméthyl cyclopropanecarboxylate de 4-(2-méthyl 2-propényl) 1-indanyle.

[alpha]_{D} = +20¤ ± 1¤ c=1% CHCl₃
Alcool utilisé voir préparation 15 .

### Exemple 44: [1R-[1alpha,3alpha(Z)]] 2,2-diméthyl 3-[3-(1,1-diméthyléthoxy) 3-oxo 1-propényl] cyclopropanecarboxylate de 4-(2-méthyl 2-propényl) 1-indanyle.

[alpha]_{D} = +48,5¤ ± 1,5¤ c=1% CHCl₃

### Exemple 45 : [1R-[1alpha(RS*),3alpha(E)]] 3-(3-éthoxy 2-fluoro 3-oxo 1-propényl) 2,2-diméthyl cyclopropanecarboxylate de 4-(2-propynyl) 1-indanyle.

[alpha]_{D} = +22,5¤ ± 2¤ c=0,5% CHCl₃
Alcool utilisé voir préparation 16 . Agr. Biol. Chem. 1978. 42. 1365.

### Exemple 46 : [1R-[1alpha(RS*),3alpha(Z)]] 3-[3-(1,1-diméthyléthoxy) 3-oxo-1-propényl] 2,2-diméthyl cyclopropanecarboxylate de 4-(2-propynyl) 1-indanyle.

[alpha]_{D} = +51¤ ± 2,5¤ c=0,5% CHCl₃

### Exemple 47 : [1R-[1alpha(RS*),3alpha(E)]] 3-[3-(1,1-diméthyléthoxy) 2-fluoro 3-oxo 1-propényl] 2,2-diméthyl cyclopropanecarboxylate de 4-(2-propynyl) 1-indanyle.

[alpha]_{D} = +30° ± 2,5° c=0,4% CHCl₃

### Exemple 48 : [1R-[1alpha(RS*),3alpha(Z)]] 3-[3(1,1-diméthyléthoxy) 3-oxo-1-propényl] 2,2-diméthyl cyclopropanecarboxylate de 4-éthènyl-1-indanyle.

[alpha]_{D} = +51,5° ± 2,5° c=0,5% CHCl₃

### Exemple 49 : [1R-[1alpha(RS*),3alpha(E)]] 3-(3-éthoxy 2-fluoro 3-oxo 1-propényl) 2,2-diméthyl cyclopropanecarboxylate de 4-éthènyl 1-indanyle

[alpha]_{D} = +17,5° ± 2,5° c=0,5% CHCl₃
L'alcool de départ est préparé selon le procédé décrit dans Agr. Biol. Chem. 1978. 42 1365.

### Exemple 50 : [1R-[1alpha(RS*),3alpha(E)]] 2,2-diméthyl 3-(2-fluoro 3-éthoxy 3-oxo 1-propényl) cyclopropanecarboxylate de 4-éthynyl 1-indanyle.

[alpha]_{D} = +17,5° ± 2° c=0,5% CHCl₃

### Exemple 51 : [1R-[1alpha(RS*),3alpha(Z)]] 3-[3-(1,1-diméthyléthoxy) 3-oxo 1-propényl] 2,2-diméthyl cyclopropanecarboxylate de 4-éthynyl 1-indanyle.

[alpha]_{D} = +50° ± 2,5° c=0,5% CHCl₃
Les alcools de départ correspondant sont préparés selon le procédé décrit dans Agr. Biol. Chem. 1978, 42 1365.

### Exemple 52 : [1R-[1alpha 3alpha(E)]] 3-[3-(1,1-diméthyléthoxy) 2-fluoro 3-oxo 1-propènyl] 2,2-diméthyl cyclopropanecarboxylate de 4-(2-chloro 2-propènyl) 1-indanyle.

[alpha]_{D} = +27° ± 3° c=0,4% CHCl₃
Alcool utilisé voir préparation 17.

### Exemple 53 : [1R-[1alpha 3alpha(E)]] 3-[3-éthoxy 2-fluoro 3-oxo 1-propènyl] 2,2-diméthyl cyclopropanecarboxylate de 4-(2-chloro 2-propényl) 1-indanyle.

[alpha]_{D} = +20° ± 1,5° c=0,8% CHCl₃

### Exemple 54: [1R-[1alpha,3alpha(Z)]] 3-[3-(1,1-diméthyléthoxy) 3-oxo 1-propènyl] 2,2-diméthyl cyclopropanecarboxylate de 4-tributylstannyl 1-indanyle.

[alpha]_{D} = +33° ± 1,5° c=1% CHCl₃

### Exemple 55 : [1R-[1alpha(RS*),3alpha(E)]] 3-éthoxy 2-fluoro 3-oxo 1-propényl) 2,2-diméthyl cyclopropanecarboxylate de 3,3-diméthyl 1-indanyle.

[alpha]_{D} = +31° c=0,3% CHCl₃

### Exemple 56 : [1R-[1alpha(RS*) 3alpha(Z)]] 3-[3-(1,1-diméthyléthoxy) 3-oxo 1-propényl] 2,2-diméthyl cyclopropanecarboxylate de 3,3-diméthyl 1-indanyle.

[alpha]_{D} = +64° c=0,4% CHCl₃

### Préparation 1 : cis (+) 2-fluoro 4-(2-propényl) 1-indanol.

### Stade A : 4-(2-propényl) 1-indanone.

On porte à 120°C pendant 1 heure 15 g de 4-bromo 1-indanone, 24 ml de tributyle allyle stannane, 150 ml de diméthylformamide, 0,82 g de tétrakis triphényl phosphine palladium et 7,89 g de triéthylamine. Après refroidissement à 20°C on verse le mélange réactionnel sur 300 ml d'une solution aqueuse de fluorure de potassium, agite, essore sur célite et extrait le filtrat à l'éther isopropylique. On reprend l'insoluble avec de l'acétate d'éthyle essore sur célite. On lave les phases organiques réunies avec de l'eau saturée en chlorure de sodium, sèche et amène à sec sous pression réduite. On chromatographie le résidu sur silice avec de l'hexane-acétate d'éthyle 9-1 et recueille 11,84 g de produit attendu.

### Spectre IR dans CHCl₃ :

| | |
|---|---|
| c = O | 1703 cm⁻¹ |
| -CH = CH₂ | 1640 cm⁻¹ |
| | 922 cm⁻¹ |
| | 996 cm⁻¹ |
| Aromatique | 1602 cm⁻¹ |
| | 1592 cm⁻¹ |
| | 1483 cm⁻¹ |

### Stade B : 3-triméthylsilyloxy 7-(2-propenyl) indène.

On refroidit à -40°C 1,24 ml de diisopropylamine et 6 ml de tétrahydrofuranne puis ajoute goutte à goutte 4,7 ml de butyllithium. On laisse la température remonter à -20°C, agite 1/4 d'heure puis introduit à -70°C 1 g de 4-(2-propényl) 1-indanone dans 10 ml de tétrahydrofuranne, agite pendant 1 heure à -60°C, ajoute 1,15 ml de chlorure de triméthysilyle dans 6 ml de tétrahydrofuranne et agite 1 heure à -60°C. On verse le mélange sur 100 ml d'eau et de glace et 100 ml d'éther. On décante, extrait la phase aqueuse à l'éther, réunit les phases organiques, les sèche et concentre à sec sans chauffer, isole 1,51 g de produit attendu.

### Stade C : 2-fluoro 4-(2-propényl 1-indanone.

On agite 5 minutes 4,58 g de produit obtenu ci-dessus dans 50 ml de chlorure de méthylène, introduit en une seule fois 6,03 g de trifluorométhyl sulfonate de N-fluoropyridine et porte au reflux pendant 5 heures. On verse le mélange réactionnel sur 250 ml d'eau et glace et 250 ml de chlorure de méthylène. On décante, lave à l'eau, extrait les phases aqueuses par du chlorure de méthylène, sèche et concentre à sec. On chromatographie le résidu sur silice élue par un mélange hexane-éther isopropylique 85-15 et obtient 2,69 g de produit attendu.

### Spectre IR CHCl₃ :

| | |
|---|---|
| C=O | 1729 cm⁻¹ |
| -CH=CH₂ | 1640 cm⁻¹ |
| | 921 cm⁻¹ |
| Aromatique | 1603 cm⁻¹ |
| | 1592 cm⁻¹ |
| | 1483 cm⁻¹ |

### Stade D : cis (+) 2-fluoro 4-(2-propényl) 1-indanol et son isomère trans (+).

On agite 5 minutes 2,62 g de produit obtenu ci-dessus dans 50 ml d'éthanol 90°, refroidit à 0°C et introduit en plusieurs fois 0,37 g de borohydrure de sodium puis agite 1 heure à 0°C. On concentre à sec sous pression réduite, reprend le résidu par 100 ml d'eau et 100 ml de chlorure de méthylène. On décante, extrait la phase aqueuse au chlorure de méthylène, sèche et concentre à sec sous pression réduite. On chromatographie le résidu sur silice dans le système hexane-éther isopropylique (7-3) et isole 0,21 g d'isomère trans brut que l'on rechromatographie pour obtenir 0,14 g d'isomère trans et 1,72 g de produit attendu (isomère cis).

### Spectre IR CHCl₃ (isomère cis) :

| | |
|---|---|
| -OH | 3590 cm⁻¹ |
| CH₂ = CH | 3080 cm⁻¹ |
| | 1639 cm⁻¹ |
| | 919 cm⁻¹ |

### Préparation 2 : [1alpha(R), 2alpha(S)] + [1alpha(S), 2alpha(R)] 2-chloro 4-(2-propényl) 1-indanol.

### Stade A : 2-chloro-4-(2-propényl) 1-indanone.

On refroidit à 0°C 8 g de 3-triméthylsilyloxy-7-(2-propényl) indène dans 25 ml de 1,2-dichloroéthane, introduit en 25 minutes 31 g de N-chlorosuccinimide dans 80 ml de dichloroéthane, agite 30 minutes à 0°C, ajoute 0,6 g de N-chlorosuccinimide dans 15 ml de dichloroéthane et quelques mg d'acide paratoluène sulfonique. Après 30 minutes d'agitation à 0°C on verse le mélange réactionnel sur une solution aqueuse glacée, saturée en phosphate monopotassique, agite 16 heures à 20°C. On décante, extrait au chlorure de méthylène, sèche, concentre à sec sous pression réduite. On chromatographie le résidu sur silice, élue par le mélange hexane éther isopropylique 7-3. On concentre les fractions recueillies et chromatographie à nouveau sur silice, élue par un mélange hexane-éther isopropylique 3-1 et isole 2,75 g de produit attendu.

### Spectre RMN 60 MHz (CDCl₃) ppm :

4,40 à 4,75 (m, système ABX) CH 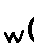 Cl
3,37 à 4,47 (m, système ABX)-CHCl-C 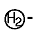 -
3,98 (d, 2H)-C 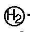 -CH = CH₂
5,62 à 6,37 (m) CH₂-C Ⓗ = CH₂
4,8 à 5,37 CH₂-CH = C 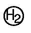
7,15 à 7,8 aromatiques

### Stade B : [1alpha(R), 2alpha(S)] + [1alpha(S), 2alpha(R)] 2-chloro 4-(2-propényl) 1-indanol.

On refroidit à 0°C 2,69 g de produit obtenu ci-dessus dans 50 ml d'éthanol, ajoute 260 mg de boronhydrure de sodium à 95 % et agite 30 minutes à 0°C et verse sur une solution aqueuse, glacée, saturée en phosphate monopotassique. On extrait à l'éther, sèche et concentre à sec sous pression réduite. On chromatographie le résidu sur silice, élue sous pression par un mélange : chlorure de méthylène-hexane-éther isopropylique 3-6-1 et recueille 2,28 g de produit attendu RS cis F = 54°C et 0,19 g de produit RS trans F = 64°C.

### Préparation 3 : (cis + trans) 2-méthyl-4-(2-propényl) 1-indanol.

### Stade A : 2-méthyl-4-(2-propényl) 1-indanone.

On refroidit à 0°C 1,88g de méthylate de sodium dans 15 ml d'éther éthylique, introduit en 15 minutes la solution composée de 2,8 ml de formiate d'éthyle 3 g de 4-(2-propenyl) indanone dans 6 ml d'éther, ajoute 5 ml d'éther, agite 15 minutes et laisse revenir la suspension à 20°C. On ajoute 70 ml de diméthyl formamide, agite 2 heures et introduit 2,15 ml d'iodure de méthyle et 2 ml d'éther puis agite 2 heures. On verse alors sur 100 ml d'eau glacée et extrait à l'éther éthylique, sèche et évapore le solvant sous pression réduite. On chromatographie le résidu sur silice, élue par un mélange hexane-acétate d'éthyle 95-5 et obtient 1,62 g de produit attendu.

### Spectre IR :

| | |
|---|---|
| c=O | 1707 cm⁻¹ |
| CH₂=CH- | 1639 cm⁻¹ |
| | 921 cm⁻¹ |
| | 990 cm⁻¹ |
| | 3085 cm⁻¹ |
| Aromatiques | 1603 cm⁻¹ |
| | 1591 cm⁻¹ |
| | 1490 cm⁻¹ |
| CH₃= | 1374 cm⁻¹ |

### Stade B : (cis + trans) 2-méthyl-4-(2-propényl) 1-indanol.

On refroidit à 0°C 1,6 g de produit obtenu en A dans 30 ml d'éthanol à 90°, introduit 170 mg de borohydrure de sodium à 95 % et laisse revenir le milieu réactionnel à 20°C. Après une heure d'agitation on ajoute 170 mg de borohydrure de sodium et agite 3 heures. On évapore l'éthanol sous pression réduite, dilue la solution aqueuse avec 10 ml d'eau et extrait au chlorure de méthylène. On sèche la phase organique, évapore le solvant sous pression réduite et chromatographie le résidu sur silice puis élue par le mélange hexane-acétate d'éthyle. On obtient 1,52 g de produit attendu (mélange).

### Spectre IR :

-OH∼ 3600 cm⁻¹

| | |
|---|---|
| CH₂=CH- | 1639 cm⁻¹ |
| | 918 cm⁻¹ |
| | 997 cm⁻¹ |
| Aromatiques | 1600 cm⁻¹ |
| | 1476 cm⁻¹ |

### Préparation 4 : (RS) 5-(2-propényl) 1-indanol.

### Stade A : 2-(3-bromobenzyl) malonate de diéthyle.

On ajoute par fraction 11,52 g d'hydrure de sodium à 50 % dans l'huile dans 120 ml de diméthylformamide et 285 ml de toluène, agite 10 minutes et refroidit à 0°C. On introduit en 15 minutes 36 ml de malonate d'éthyle dans 36 ml de toluène et agite 1 heure à 0° + 5°C. Dans 30 g de bromure de 3-bromobenzyle et 150 ml de toluène on ajoute goutte à goutte durant 2 heures 15 minutes à 20°C le mélange réactionnel obtenu ci-dessus et agite pendant 4 heures puis verse le milieu dans 500 ml d'eau. On extrait avec de l'éther isopropylique, lave la phase organique avec 240 ml de l'acide chlorhydrique N, jusqu'à pH 4 puis avec de l'eau jusqu'à pH 7. On sèche, filtre et amène à sec le filtrat sous pression réduite. On chromatographie le résidu sur silice élue par un mélange hexane-acétate d'éthyle 9-1 et obtient 24,07 g de produit attendu et un produit dibromobenzylé.

### Spectre IR CHCl₃ :

| | |
|---|---|
| C=O | 1745 cm⁻¹ |
| | 1730 cm⁻¹ |
| Aromatiques | 1599 cm⁻¹ |
| | 1570 cm⁻¹ |
| | 1479 cm⁻¹ |

### Stade B : Acide 2-(3-bromobenzyl) malonique.

On porte au reflux durant 5 heures 30 minutes 25 g de produit obtenu ci-dessus et 25 g de potasse dans 25 ml d'eau. On reprend le précipité par 300 ml d'eau, lave à l'éther isopropylique et ajoute à la phase aqueuse tout en refroidissant 60 ml d'acide chlorhydrique concentré jusqu'à pH 1. On extrait à l'éther isopropylique, lave à l'eau, sèche et amène à sec sous pression réduite. On reprend le résidu par 200 ml d'hexane, agite 1 heure, filtre, sèche et récupère 18,85 g de produit attendu. F = 117°C.

### Stade C : Acide 3-(3-bromophényl) propionique.

On porte au reflux 18,85 g de produit obtenu ci-dessus dans 75 ml d'eau pendant 18 heures. On extrait à l'éther isopropylique, sèche, amène à sec sous pression réduite et obtient 15,75 g de produit attendu. F = 74°C.

### Stade D : 5-bromo 1-indanone et 7-bromo 1-indanone.

On porte au reflux durant 1 heure 30 minutes 15,7 g de produit obtenu en C et 80 ml de chlorure de thionyle et distille le chlorure de thionyle en excès sous pression réduite. On ajoute 300 ml de chlorure de méthylène et 11,5 g de chlorure d'aluminium puis porte au reflux durant 3 heures. On dilue avec 200 ml d'acide chlorhydrique N et de la glace extrait au chlorure de méthylène, lave la phase organique jusqu'à pH 7 avec de l'eau, sèche amène à sec sous pression réduite. On chromatographie le résidu sur silice, élue au chlorure de méthylène et recueille 2,09 g de 7-bromo 1-indanone, 1,48 g de mélange des indanones et 9,21 g de 5-bromo 1-indanone. On chromatographie le mélange sur silice, élue par un mélange hexane-acétate d'éthyle (8-2) et sépare 0,75 g de 5-bromo 1-indanone et 0,66 g de 7-bromo 1-indanone.
5-bromo 1-indanone F = 130°C
7-bromo 1-indanone F = 114°C.

### Stade E : 5-bromo 1-indanol (racémique).

On dissout 1,5 g du produit obtenu au stade D dans 15 ml d'éthanol anhydre et 5 ml de tétrahydrofuranne, ajoute 0,19 g d'hydroborure de sodium à 95 % et agite 1 heure 30 minutes à 20°C puis amène à sec sous pression réduite. On reprend le résidu avec 100 ml d'eau contenant du chlorure de sodium et 100 ml de chlorure de méthylène. On décante, extrait au chlorure de méthylène, lave à l'eau saturée en chlorure de sodium, sèche et amène à sec sous pression réduite. On obtient 1,52 g de produit attendu F = 73°C.

### Stade F : 5-(2-propényl) 1-indanol (racémique).

On porte à 140°C durant 45 minutes 4,67 g de produit obtenu ci-dessus, 46 ml de diméthylformamide, 7,98 g de tributylallylstannane et 1,39 g de tétrakis triphényl phosphine palladium. Après refroidissement on verse le mélange réactionnel dans 200 ml d'eau contenant du fluorure de potassium, agite 15 minutes, essore et rince à l'acétate d'éthyle. On extrait le filtrat à l'acétate d'éthyle lave à l'eau, sèche et amène à sec sous pression réduite. On chromatographie le résidu sur silice dans le système hexane-acétate d'éthyle 7-3 et obtient 2,92 g de produit attendu que l'on reprend par 30 ml de pentane, agite 1 heure, filtre, sèche à 45° et récupère 2,55 g de produit F = 54°C.

### Préparation 5 : 6-(2-propényl) 1-indanol.

On opère comme dans la préparation 6 en partant du bromure de 4-bromobenzyle et obtient le produit attendu.

### Spectre IR CHCl₃ :

| | |
|---|---|
| OH | 3597 cm⁻¹ |
| CH₂=CH | 3080 cm⁻¹ |
| | 1639 cm⁻¹ |
| | 918 cm⁻¹ |
| | 996 cm⁻¹ |

### Préparation 6 : 1-indanol (racémique).

### Stade A : 1-indanone.

On opère comme à la préparation 8 à partir de 2,8 g de 4-bromo 1-indanone et obtient 1,28 g de produit attendu.

### Spectre IR dans CHCl₃ :

| | |
|---|---|
| C = O | 1708 cm⁻¹ |
| Aromatique | 1610 (max) |
| | 1600 (ep), 1592 (ep) |

### Stade B: 1-indanol (racémique).

On refroidit à +5°C 1,28 g de 1-indanone dans 13 ml d'éthanol anhydre. On ajoute par petites fractions 0,23 g de borohydrure de sodium à 95 % agite à +10°C pendant 1 heure. A +5°C on ajoute à nouveau 0,23 g de borohydrure de sodium à 95 % et agite à 20°C durant 1 heure 30 minutes. On verse le mélange réactionnel dans 60 ml d'eau contenant du chlorure de sodium et extrait à l'éther isopropylique, sèche, amène à sec sous pression réduite et chromatographie le résidu sur silice dans le système hexane-acétate d'éthyle 6-4. On isole 1,15 g de produit attendu.

### Spectre IR CHCl₃ :

| | |
|---|---|
| OH | = 3600 cm⁻¹ |
| | 1478 cm⁻¹ |

### Préparation 7 : 1-hydroxy 4-indanecarbonitrile (racémique).

On refroidit à +5°C 1,46 g de 4-cyano-1-indanone dans 131 ml de tétrahydrofuranne, ajoute par petites fractions 1 g de borohydrure de potassium, agite pendant 1 heure 15 minutes et verse sur 200 ml d'eau contenant du chlorure de sodium puis extrait à l'éther isopropylique. On sèche la phase organique, amène à sec sous pression réduite et chromatographie le résidu sur silice dans un mélange hexane-acétate d'éthyle 6-4. On obtient 1,40 g de produit attendu F = 98°C.

### Préparation 8 : (RS) 1-hydroxy 5-indanecarbonitrile.

On opère comme à la préparation 10 à partir de la 5-cyano 1-indanone (1,93 g) reprend le résidu d'extraction par 20 ml d'hexane, agite 1 heure, filtre, sèche à 45°C sous pression réduite et obtient 1,92 g F = 94°C.

### Préparation 9 : 1-hydroxy 4-indanecarbonitrile R(+) et 1-hydroxy 4-indanecarbonitrile S(-).

### Stade A : acétate de (4-cyano 1-indanyle) racémique.

On refroidit à 0°C 3,89 g de (RS) 1-hydroxy 4-indanecarbonitrile dans 39 ml de pyridine, ajoute goutte à goutte 3,45 ml d'anhydride acétique et agite pendant 16 heures à 20°C. On verse le mélange réactionnel dans 300 ml d'eau contenant du chlorure de sodium, extrait à l'éther isopropylique, lave la phase éthérée à l'acide chlorhydrique 2N puis à l'eau jusqu'à pH 7. On sèche, amène à sec la phase éthérée, reprend le résidu avec du 1,2-dichloroéthane, concentre à nouveau à sec sous pression réduite. On reprend le résidu par 40 ml d'hexane, agite 1 heure, filtre et récupère 4,46 g de produit attendu. F = 65°C.

### Stade B : R(+) 1-hydroxy 4-indanecarbonitrile et son acétate S(-).

On agite à 20°C 4,46 g de produit obtenu ci-dessus 24,4 ml d'acétone et 190 ml de tampon pH 8. On contrôle le pH soit 7,98 et introduit 4,46 g d'enzyme (PPL Sigma type II : lipase du pancréas de porc) on agite 28 heures à 20°C, verse dans 200 ml d'acide chlorhydrique 2N et 200 ml d'acétate d'éthyle, ajoute de la célite (clarcel) agite de nouveau durant 15 minutes. On filtre sur célite, rince, décante et extrait à l'acétate d'éthyle, sèche et amène à sec sous pression réduite. On chromatographie le résidu sur silice dans le système hexane-acétate d'éthyle (7-3). On obtient 1,05 g d'alcool (R) attendu, F = 98°C, [alpha]_{D} : +13° ± 2 (c = 0,6 % CHCl₃) et 2,96 g d'acétate riche en isomère (S). On agite ces 2,96 g de produit dans 16,2 ml d'acétone et 120 ml de tampon pH 8, ajuste le pH à 8 avec 2 ml d'acide chlorhydrique 1N et introduit en une seule fois 2,96 g d'enzyme puis agite pendant 19 heures. On verse le mélange réactionnel dans 150 ml d'acide chlorhydrique 2N et 150 ml d'acétate d'éthyle, agite 5 minutes, ajoute de la célite et agite 15 minutes. On filtre sur célite, rince, décante et extrait à l'acétate d'éthyle. On sèche la phase organique, concentre à sec sous pression réduite, chromatogra-phie le résidu dans le système hexane-acétate d'ethyle 7-3 et obtient 2,22 g d'acétate de S(-) 4-cyano 1-indanyle et 0,49 g d'alcool (R) F= 98°C.

### Stade C : S(-) 1-hydroxy 4-indanecarbonitrile.

On agite 2,12 g d'acétate obtenu ci-dessus dans 21 ml d'alcool à 90 %, agite 5 minutes, ajoute 1,36 ml de soude concentrée et agite 15 minutes à 0°C et 15 minutes à 10°C. On verse le mélange réactionnel dans 50 ml d'eau contenant de l'hydrogénophosphate de potassium extrait au chlorure de méthylène, sèche la phase organique et amène à sec sous pression réduite. On reprend le résidu avec 17 ml d'hexane, agite 1 heure, filtre, sèche à 45°C sous pression réduite et récupère 1,56 g de produit attendu F = 98°C.
[Alpha]_{D} = -14° ± 1° (c = 1 % CHCl₃).

### Préparation 10 : 1-hydroxy 6-indanecarbonitrile racémique.

### Stade A : 1-oxo 6-indanecarbonitrile.

On porte au reflux 2 g de 6-bromo-indanone, 10 ml de diméthylformamide et 3 g de cyanure de cuivre pendant 20 heures. A 20°C on verse le mélange réactionnel dans 40 ml d'eau et 30 ml de chlorure de méthylène, agite 15 minutes, filtre sur célite, décante le filtrat et réextrait avec du chlorure de méthylène. On lave à l'eau la phase organique, sèche, amène à sec sous pression réduite et chromatographie le résidu sur silice dans le mélange éluant hexane-acétate d'éthyle 7-3. On obtient 1,03 g de produit attendu F = 109°C.

### Stade B : 1-hydroxy 6-indanecarbonitrile racémique.

On refroidit à 0°C - 5°C 1 g de produit obtenu ci-dessus 100 ml de tétrahydrofuranne et 15 ml d'eau et ajoute 950 mg de borohydrure de potassium à 95 % puis agite 10 minutes à 0°C et 1 heure à 20°C. On verse le mélange réactionnel sur 150 ml d'eau contenant du chlorure de sodium, décante la phase aqueuse extrait à l'éther isopropylique, sèche et concentre à sec sous pression réduite. On chromatographie le résidu sur silice dans le système hexane-acétate d'éthyle (6-4) et obtient 980 mg de produit attendu.

### Préparation 11 : 1-hydroxy 7-indanecarbonitrile racémique.

On opère comme à la préparation 14 à partir de 1,84 g de 7-bromoindanone et obtient 1,07 g de produit attendu F = 73°C.

### Préparation 12 : 1-hydroxy 4-indaneacétonitrile.

### Stade A : alpha-bromo 2-iodo toluène.

On mélange 22 g de 2-iodotoluène, 100 ml de tétrachlorure de carbone, 19,67 g de N-bromo succinimide et 1 g de peroxyde de benzoyle et chauffe au reflux pendant 8 heures. On filtre, rince l'insoluble au tétrachlorure de carbone et distille le filtrat d'abord sous pression atmosphérique puis sous pression réduite. On obtient le produit attendu utilisé tel quel pour la suite de la synthèse.

### Stade B : 4-iodo 1-indanol.

On opère comme à la préparation 6 Stade B, C, D et E à partir du produit obtenu ci-dessus pour obtenir le produit attendu F = 83-84°C.

### Stade C : 4-iodo 1-triméthylsilyloxy indane racémique.

On amène à 0°C 5,2 g de l'alcool obtenu ci-dessus dans 100 ml d'éther et 5 ml de triéthylamine et ajoute 4,1 ml de chlorure de triméthyl silyle puis agite 1 heure à température ambiante. On filtre, rince l'insoluble avec de l'éther et ajoute de l'eau au filtrat. On décante, extrait à l'éther, lave à l'eau jusqu'à neutralité, sèche et évapore à sec. On obtient 5,9 g de produit brut utilisé tel quel.

### Stade D : 1-hydroxy 4-indaneacétique.

On refroidit à +15°C, 20 ml de 1,3-diméthyl imidazolidone et 3,34 g d'hydrure de sodium à 50 %. On ajoute en dix minutes 7 ml de cyano acétate d'éthyle dans 33 ml de 1,3-diméthyl imidazolidone et agite 1 heure. On ajoute à la solution obtenue, 13,2 g d'iodure cuivreux, 11,5 g de dérivé iodé obtenu en C et 11 ml de 1,3-diméthyl imidazolidone puis porte à 95°C pendant 4 heures. A la suspension obtenue, on ajoute en 5 minutes, à 90°C 2,5 g de soude dans 52 ml d'eau et maintient le chauffage à 90°C durant 2 heures. Après refroidissment, on verse le milieu dans 400 ml d'acide chlorhydrique 2N, agite 30 minutes, essore sur célite, rince à l'eau et à l'acétate d'éthyle. On décante, sature la phase aqueuse avec du chlorure de sodium et extrait à l'acétate d'éthyle. On lave les phases organiques avec une solution saturée en bicarbonate de sodium à l'eau saturée en chlorure de sodium, sèche et amène à sec sous pression réduite. On chromatographie le résidu sur silice avec un système (dichloro difluoro éthane)-acétate d'éthyle (1-1) et isole 4,36 g de produit brut. On reprend à chaud ce dernier par 180 ml d'éther isopropylique, filtre et récupère 1,05 g de produit attendu (cristaux blancs) et 3,24 g de cristaux beiges que l'on rechromatographie pour obtenir 1,84 g de produit attendu et 0,79 g de produit attendu impur.

### Spectre IR dans CHCl₃ :

| | |
|---|---|
| Présence OH | 3599 cm⁻¹ |
| CN | 2255 cm⁻¹, 210 cm⁻¹ |
| Aromatiques | 1616 cm⁻¹ 1600 cm⁻¹, 1479 cm⁻¹. |

### Préparation 13 : alpha-cyano 1-hydroxy 4-indaneacétate d'éthyle.

On refroidit à +15°C 6 ml de 1,3-diméthyl 2-imidazolidone et 0,99 g d'hydrure de sodium à 50 %, ajoute en 5 minutes 2,2 ml de cyanacétate d'éthyle dans 10 ml de 1,3-diméthyl 2-imidazolidone et agite durant 40 minutes. On ajoute 4,39 g d'iodure cuivreux, 3,62 g de 4-iodo 1-triméthylsilyloxy indane et 4 ml de 1,3-diméthyl 2-imidazolidone. On porte le mélange à 100°C durant 4 heures. Après refroidissement on le verse dans 100 ml d'acide chlorhydrique 2N, essore sur célite, rince à l'eau et extrait le filtrat à l'éther isopropylique, lave avec une solution saturée de bicarbonate de soude, à l'eau sèche et amène à sec. On chromatographie le résidu sur silice dans un mélange hexaneacétate d'éthyle (1-1) et obtient 1,22 g de produit attendu.

### Spectre IR CHCl₃ :

| | |
|---|---|
| - OH | 3600 cm⁻¹ |
| - CN | 2255 cm⁻¹ |
| - C = O | 1746 cm⁻¹ |

### Préparation 14 : 4-propyl 1-indanol racémique.

### Stade A : 4-propyl 1-indanone.

On dissout 1,63 g de 4-(2-propényl) 1-indanone dans 300 ml d'éthanol anhydre 400 mg de rhodium à 5 % sur charbon actif et hydrogène à température et pression ambiante pendant 3/4 heure. On filtre sur célite, lave à l'éthanol et évapore à sec le filtrat et obtient 1,56 g de produit attendu.

### Stade B : 4-propyl 1-indanol racémique.

On refroidit à 0°C 1,56 g de produit ci-dessus dans 30 ml d'éthanol, ajoute 0,2 g de borohydrure de sodium et laisse 16 heures à température ambiante. On verse sur 300 ml d'eau et extrait à l'éther, sèche et évapore à sec sous pression réduite. On obtient 1,58 g de produit attendu.

### Spectre IR :

plus de C = O
OH 3600 cm⁻¹

### Préparation 15 : 4-(2-méthyl 2-propényl) 1-indanol.

### Stade A : 4-(2-méthyl 2-propényl) 1-indanone.

On chauffe à 125°C 9 g de (2-méthyl 2-propényl) tributyl stannane, 5,25g de 4-bromo indanone, 50 ml de diméthyl formamide et 300 g de tétrakis phényl phosphine palladium sous agitation pendant 45 minutes. Après refroidissement on verse sur une solution glacée de 7,5 g de fluorure de potassium dans 300 ml d'eau, agite 5 minutes et filtre sur célite. On extrait le filtrat à l'éther isopropylique sèche et concentre à sec sous pression réduite. On chromatographie sur silice le résidu, élue par le mélange hexane-acétate d'éthyle 8-2 et recueille 4 g de produit attendu.

### Spectre IR :

| | |
|---|---|
| C = O | 1706 cm⁻¹ |
| C = CH₂ | 1649 cm⁻¹ |
| | 897 cm⁻¹ |
| Aromatique | 1603 cm⁻¹ |
| | 1592 cm⁻¹ |
| | 1482 cm⁻¹ |

### Préparation du (2-méthyl-2-propényl) tributyl stannane

On mélange 4,7 g de tournures de magnésium dans 15 ml de tétrahydrofuranne, ajoute 5 gouttes de dibromo-éthane et 1 goutte de chlorure de méthallyle. Lorsque la réaction a démarré, on porte au reflux tout en introduisant lentement une solution contenant 10 ml de chlorure de méthallyle 16,3 ml de chlorure de tributyl-étain et 50 ml de térahydrofuranne. Après 12 heures au reflux, on refroidit verse le mélange réactionnel sous atomosphère inerte sur une solution glacée, saturée en chlorure d'ammonium (30 ml), filtre sur célite, rince avec 30 ml d'une solution saturée de chlorure de sodium et extrait le filtrat à l'éther éthylique. On lave les phases organiques réunies avec 30 ml d'une solution de phosphate monopotassique, puis 30 ml d'eau. Après sèchage on évapore le solvant sous pression réduite et obtient 18,8 g de produit brut. On rectifie celui-ci sous 0,2 mm Hg et recueille 17,5 g eb_{2mmHg} 83-93°C.

### Spectre IR :

| | |
|---|---|
| - C = CH₂ | 3080 cm⁻¹ |
| | 1627 cm⁻¹ |
| | def 864 cm⁻¹ |

### Stade B : 4-(2-méthyl 2-propényl) 1-indanol.

On refroidit à 0°C 4,2 g de produit obtenu ci-dessus dans 80 ml d'éthanol, ajoute par fractions 1,350 g de borohydrure de sodium et agite pendant 2 heures. On verse le mélange réactionnel sur 150 ml d'une solution glacée de phosphate monopotassique, extrait à l'ether, sèche et évapore le solvant sous pression réduite. On chromatographie le résidu sur silice, élue par un mélange hexane-acétate d'éthyle (8-2) et recueille 3,95 g de produit attendu.

### Spectre IR :

| | |
|---|---|
| - OH | 3600 cm⁻¹ |
| C = CH₂ | 1649 cm⁻¹ |
| | 895 cm⁻¹ |
| Aromatique | 1600 cm⁻¹ |
| | 1479 cm⁻¹ |

### Préparation 16 : 4-(2-propenyl) 1-indanol RS et son isomère 2-ol.

On chauffe à 65°C 0,61 g de tournures de magnésium dans 1 ml de tétrahydrofuranne, ajoute 2 gouttes de 1,2-dibromoéthane et, d'abord 2 gouttes, puis goutte à goutte une solution de 7 g de 4-bromo 1-triméthyl siloxy indane dans 41 ml de tétrahydrofuranne. Ensuite on porte au reflux pendant 2 heures et obtient un magnésien titrant 0,56N. On agite 1,7 g de méthoxyallène, 0,28 g de iodure cuivreux dans 24 ml d'éther et ajoute goutte à goutte 44 ml du magnésien en 10 minutes. Après 45 minutes on ajoute 1 g de méthoxyallène et agite encore 35 minutes à 20°C. On verse le milieu réactionnel dans 150 ml d'une solution saturée en chlorure d'ammonium, ajoute 2 ml d'une solution d'ammoniaque à 20 % et extrait à l'éther isopropylique. On lave à l'eau la phase étherée, puis avec une solution 0,2 N de thiosulfate de sodium et enfin à l'eau. On sèche et amène à sec sous pression réduite. On reprend le résidu huileux obtenu par 25 ml de tétrahydrofuranne, ajoute d'un coup 16,2 ml d'une solution 1M de fluorure de tétrabutylammonium dans le tétrahydrofuranne et agite 10 minutes. On verse le milieu réactionnel dans 100 ml d'eau, extrait à l'éther isopropylique lave la phase étherée, à l'eau jusqu'à pH 7, sèche et amène à sec sous pression réduite. On chromatographie le résidu sur silice dans un mélange hexane-acétate d'ethyle (7-3) et recueille 1,56 g de produit attendu F = 70°C.

### Spectre IR CHCl₃ :

| | | |
|---|---|---|
| OH 3600 cm⁻¹ | | |
| C≡CH | ( ≡CH) | 3310 cm⁻¹ |
| | (C≡C)∼ | 2100 cm⁻¹ |
| Aromatique | | 1599 cm⁻¹ |
| | | 1478 cm⁻¹ |

En partant du 4-bromo 2-triméthylsiloxy indane et en opérant comme ci-dessus on obtient le 2,3-dihydro 4-(2-propenyl) 2H inden 2-ol.

### Préparation 17: 4-(2-chloro-2-propenyl) 1-indanol racémique.

### Stade A : 4-(tributyl stannyl) 1-indanone.

On dissout 1,5 g de 4-iodo indane 1-one dans 15 ml de diméthylformamide et 10 ml d'hexaméthyl phosphorotriamide, ajoute 4,35 ml d'hexabutyl di-étain 30 mg de dichloro diacetonitrile-palladium et agite 16 heures à température ambiante. On verse le mélange réactionnel dans 200 ml d'une aqueuse de fluorure de potassium, agite, filtre sur célite, rince à l'acétate d'éthyle, extrait le filtrat à l'éther isopropylique, sèche les phases organiques et concentre à sec. On chromatographie le résidu sur silice avec un mélange hexane-éther isopropylique (9-1) et isole 1,91 g de produit attendu.

### Spectre IR (CHCl₃) :

C = O 1704 cm⁻¹

### Stade B : 4-(2-chloro 2-propényl) 1-indanone.

On chauffe à 100°C 12 g de produit obtenu ci-dessus 5,25 ml de 2,3-dichloro-1-propène, 120 ml de toluène et 150 mg de dichloro-diacétonitrile-palladium et agite 20 minutes à 100°C. Après refroidissement on verse le milieu réactionnel sur 150 ml d'une solution aqueuse de 10 g de fluorure de potassium, ajoute 100 ml d'acétate d'éthyle et agite violemment pendant 10 minutes. On filtre sur célite, rince abondamment à l'acétate d'éthyle. Après décantation on réextrait à l'actétate d'éthyle, sèche les phases organiques réunies, évapore sous pression réduite. On chromatographie le résidu sur silice, élue par le mélange hexane-acétate d'éthyle 8-2 et recueille 2,83 g de produit attendu.

### Spectre IR :

| | |
|---|---|
| C = O | 1708 cm⁻¹ |
| 〉C = CH₂ | 1635 cm⁻¹ |
| | 889 cm⁻¹ |
| Aromatique | 1607 cm⁻¹ |
| | 1592 cm⁻¹ |
| | 1482 cm⁻¹ |

### Stade C : 4-(2-chloro 2-propényl) 1-indanol racémique.

On refroidit à 0°C 2,8 g de produit obtenu ci-dessus dans 60 ml d'éthanol à 90°C, introduit 250 mg de borohydrate de sodium et agite 75 minutes à 0°C. On rajoute à 20°C 250 mg de borohydrure de sodium verse sur une solution glacée de phosphate monopotassique de potassium, extrait à l'éther, sèche les phases organiques et concentre à sec sous pression réduite. On chromatographie le résidu sur silice, élue par le mélange hexane-acétate d'éthyle 3-1 et recueille 1,67 g de produit attendu.

### Spectre IR :

| | |
|---|---|
| OH | 3600 cm⁻¹ |
| 〉C = CH₂ | 1635 cm⁻¹ |
| | 890 cm⁻¹ |
| Aromatique | 1602 cm⁻¹ |
| | 1479 cm⁻¹ |

### Préparation 18 : R (-) 4-(2-propényl) 1-indanol et son isomère S (+).

### Stade A : Acétate de 4-bromo 1-indanyle.

On refroidit à 0°C 3,0 g de 4-bromo-1-indanol (RS) dans 30 ml de pyridine, introduit lentement 2 ml d'anhydride acétique et agite 17 heures à température ambiante. On verse sur 100 ml d'eau et 100 ml d'éther isopropylique, dépante lave la phase organique à l'eau, sèche et concentre à sec. On reprend le résidu par du 1,2-dichloro éthane pour éliminer la pyridine par azéotropie. On obtient 3,9 g de produit attendu.

### Spectre RMN (CDCl₃) :

2,02 ppm CH₃
2,68 ppm Ø CH₂ - CH₂
6,82 ppm 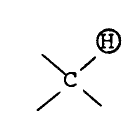
7,22 ppm (m) aromatiques

### Stade B : R (+) 4-bromo 1-indanol.

On ajuste à pH 8 par addition d'acide chlorhydrique N, 4,49 g de produit obtenu ci-dessus dans 19 ml d'acétone et 180 ml d'une solution tampon pH 8. On introduit en une fois 4,5 g de porcine pancréas lipase Sigma type II. Après 5 heures on verse le mélange réactionnel sur 200 ml d'acide chlorhydrique 2 N et 200 ml de chlorure de méthylène, ajoute de la célite et agite 1/2 heure à température ambiante. On filtre sur célite, décante le filtrat et extrait la phase aqueuse au chlorure de méthylène. On réunit les phases organiques, sèche et concentre à sec sous pression réduite. On chromatographie le résidu sur silice dans le système hexane-acétate d'éthyle 8-2 et obtient 2,38 g d'acétate riche en isomère "S" et 1,48 g d'alcool attendu "R" F = 84°C
[Alpha]_{D} + 2° ± 0,5° c = 1 % CHCl₃

### Stade C : (S) acétate de 4-bromo 1-indanyle.

On opère comme ci-dessus à partir de 2,28 g de mélange S obtenu ci-dessus, chromatographie le résidu sur silice dans le système hexane-éther-isopropylique 9-1 puis hexane-acétate d'éthyle et obtient d'abord 1,85 g d'acétate S attendu puis 0,22 g de cristaux d'alcool (mélange R + S).

### Stade D : S(-) 4-bromo 1-indanol.

On dissout 1,85 g d'acétate S obtenu ci-dessus dans 15 ml d'alcool 90 %, agite, introduit peu à peu 0,53 g de potasse en pastilles, maintient l'agitation à température ambiante pendant 1 heure et verse sur 150 ml d'eau et 100 ml de chlorure de méthylène. On décante, extrait la phase aqueuse au chlorure de méthylène lave à l'eau les phases organiques réunies, sèche et concentre à sec sous pression réduite. On obtient 1,53 g de produit attendu F = 81°C.
[Alpha]_{D} -8° ± 1° (c = 1 % CHCl₃)

### Stade E : S(+) 4-(2-propényl) 1-indanol.

On porte à 120°C pendant 2 heures 1,48 g d'alcool obtenu en D, 15 ml de diméthylformamide, 2,3 ml de tributylallyl-stannane et 80 mg de tétrakis triphényl phosphine-palladium. On verse le mélange réactionnel sur 1 g de fluorure de potassium dans 100 ml d'eau agite 15 minutes, filtre sur célite, rince à l'éther isopropylique et décante le filtrat. On lave la phase organique à l'eau la sèche et concentre à sec sous pression réduite. On chromatographie le résidu dans le système hexane acétate d'éthyle (8-2) et obtient 0,84 g de produit attendu et 0,27 g de mélange.
[Alpha]_{D} + 95° ± 2° (c = 0,6 % CHCl₃).

### Stade F : R(-) 4-(2-propényl) 1-indanol.

On opère comme au Stade E à partir de 1,37 g d'alcool R obtenu en B et obtient 1,02 g de produit attendu.
[Alpha]_{D} -12,5° ± 2° (c = 0,5° CHCl₃).

### Exemple 57: préparation d'un concentré soluble

On effectue un mélange homogène de :

| | |
|---|---|
| Produit de l'exemple 1 | 0,25 g |
| Butoxyde de pipéronyle | 1,00 g |
| Tween 80 | 0,25 g |
| Topanol A | 0,1 g |
| Eau | 98,4 g |

### Exemple 58: préparation d'un concentré émulsifiable

On mélange intimement :

| | |
|---|---|
| Produit de l'exemple 2 | 0,015 g |
| Butoxyde de pipéronyle | 0,5 g |
| Topanol A | 0,1 g |
| Tween 80 | 3,5 g |
| Xylène | 95,885 g |

### Exemple 59 : préparation d'un concentré émulsifiable

On effectue un mélange homogène de :

| | |
|---|---|
| Produit de l'exemple 54 | 1,5 g |
| Tween 80 | 20,00 g |
| Topanol A | 0,1 g |
| Xylène | 78,4 g |

### Exemple 60 : préparation d'une composition fumigène

On mélange d' une façon homogène :

| | |
|---|---|
| Produit de l'exemple 56 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-nitrophénol | 0,5 g |

### ETUDE BIOLOGIQUE

### A. Etude de l'effet d'abattage sur mouche domestique

Les insectes tests sont des mouches domestiques femelles âgées de 4 jours. On opère par pulvérisation directe à la concentration indiquée, en chambre de Kearns et March, en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés de l'exemple | KT₅₀ en mn |
|---|---|
| 1 | 5,5 à 1 g/l |
| 2 | 3,7 à 1 g/l |
| 4 | 2,5 à 1 g/l |
| 25 | 3,5 à 0,1 g/l |
| 26 | 2,4 à 1 g/l |
| 45 | 3,12 à 0,1 g/l |

### Activité sur Tétranychus urticae. Essai adulticide.

On utilise des plants de haricot comportant deux feuilles cotylédonaires. Ces plants sont traités au pistolet Fisher avec une solution acétonique du produit. Après séchage, 25 femelles de l'acarien Tétranychus urticae sont disposées par feuilles soit 50 individus par dose expérimentée par plant. Le contrôle d'efficacité est effectué après 80 heures de contact. On mesure la CL 50 en mg/hl.

| Exemple | CL 50 |
|---|---|
| 1 | 27,9 |
| 2 | 95 |
| 6 | 26,4 |
| 4 | 90 |
| 5 | 25 |
| 26 | 36,9 |

### Conclusion : Les produits de l'invention et sont doués d'un remarquable effet acaricide sur Tétranychus urticae.

### B. Etude de l'effet létal des composés de l'invention sur divers insectes Etude de l'effet létal sur mouche domestique

Les insectes tests sont des mouches domestiques femelles âgées de 4 à 5 jours. On opère par application topique de 1 ul de solution acétonique sur le thorax dorsal des insectes à l'aide du micro manipulateur d'Arnold. On utilise 50 individus par traitement. On effectue le contrôle de mortalité vingt-quatre heures après traitement.

Les résultats obtenus exprimés en DL 50 ou dose (en nanogrammes) par individu nécessaire pour tuer 50 % des insectes, sont les suivants :

| Composés de l'exemple | DL en ng/insecte |
|---|---|
| 2 | 4,2 |
| 4 | 3,9 |
| 45 | 1,7 |

### C) Etude de l'effet létal sur blatte

Les tests sont effectués par contact sur film sur verre, par dépôt à la pipette, de solutions acétoniques de différentes concentrations sur fond de boîte de Petri en verre dont les bords ont été préalablement talqués afin d'éviter la fuite des insectes. On détermine la concentration létale 50 (CL 50).

Les résultats expérimentaux obtenus sont résumés dans le tableau suivant :

| Composés de l'exemple | CL 50 en mg/m2 |
|---|---|
| 1 | 0,29 |
| 3 | 0,24 |

## Revendications

1. Sous toutes les formes isomères possibles, ainsi que leurs mélanges, les composés de formule (I) : 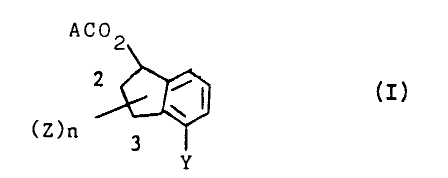 dans laquelle A représente le radical : 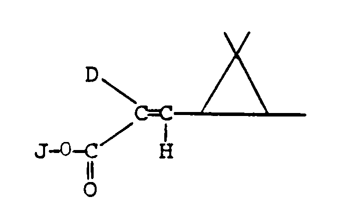 dans lequel D représente un atome d'hydrogène ou de fluor , et J représente un radical alkyle linéaire, ramifié ou cyclique, saturé ou insaturé, renfermant jusqu'à 4 atomes de carbone éventuellement substitué par un ou plusieurs atomes d'halogène ,
- Z en position 2, ou 3 représente un atome d'hydrogène, un atome de fluor, un radical alkyle linéaire, ramifié ou cyclique, saturé ou insaturé renfermant jusqu'à 8 atomes de carbone, n représentant le nombre 1, 2 ou 3 ;
- Y représente le radical -C≡N, CH₂CH=CH₂, CH₂-C≡CH ou -CH₂-C≡N.

2. Les composés de formule (I) tels que définis à la revendication 1, dont les noms suivent :
- le [1R-[1alpha(RS*),3alpha(E)]] 3-(3-éthoxy 2-fluoro 3-oxo 1-propényl) 2,2-diméthyl cyclopropanecarboxylate de 4-(2-propényl) 1-indanyle.
- le [1R-[1alpha(RS*),3alpha(Z)]] 3-[3-(1,1-diméthyléthoxy) 3-oxo 1-propényl 2,2-diméthyl cyclopropanecarboxylate de 4-(2-propényl) 1-indanyle.
- le [1R-[1alpha,3alpha(E)]] 3-[3-(1,1-diméthyléthoxy) 2-fluoro 3-oxo 1-propényl] 2,2-diméthyl cyclopropanecarboxylate de 4-(2-propényl) 1-indanyle.
- le [1R-[1alpha(R*),3alpha(E)]] 3-(3-éthoxy 2-fluoro 3-oxo 1-propényl) 2,2-diméthyl cyclopropanecarboxylate de 4-(2-propényl 1-indanyle.
- le [1R-[1alpha(R*),3alpha(E)]] 3-[3-(1,1-diméthyléthoxy) 2-fluoro 3-oxo 1-propényl] 2,2-diméthyl cyclopropanecarboxylate de 4-(2-propényl) 1-indanyle.
- le [1R-[1alpha(RS*),3alpha(E)]] 3-(3-éthoxy 2-fluoro 3-oxo 1-propényl) 2,2-diméthyl cyclopropanecarboxylate de 4-cyano 1-indanyle.
- le [1R-[1alpha(RS*),3alpha(E)]] 3-[3-(1,1-diméthyléthoxy) 2-fluoro 3-oxo 1-propényl] 2,2-diméthyl cyclopropanecarboxylate de 4-cyano 1-indanyle.
- le [1R-[1alpha(RS*),3alpha(E)]] 3-(éthoxy 2-fluoro 3-oxo 1-propényl) 2,2-diméthyl cyclopropanecarboxylate de 4-(2-propynyl) 1-indanyle.

3. Les composés de formule (I) tels que définis à l'une quelconque des revendications 1 et 2, pour leur utilisation à la lutte contre les parasites des végétaux, les parasites des locaux et les parasites des animaux à sang chaud.

4. Les compositions pesticides destinées à la lutte contre les parasites des végétaux, les parasites des locaux et les parasites des animaux à sang chaud, caractérisées en ce qu'elles renferment comme principe actif au moins un composé défini à l'une quelconque des revendications 1 et 2.

5. Les compositions insecticides renfermant comme principe actif, au moins l'un des composés définis à l'une quelconque des revendications 1 et 2.

6. Les compositions acaricides destinées à lutter contre les acariens parasites des végétaux, renfermant comme principe actif au moins l'un des composés définis à l'une quelconque des revendications 1 et 2.

7. Les compositions nématicides renfermant comme principe actif, au moins l'un des composés définis à l'une quelconque des revendications 1 et 2.

8. Les compositions acaricides destinées à lutter contre les acariens parasites des animaux, renfermant comme principe actif au moins l'un des composés définis à l'une quelconque des revendications 1 et 2.

9. Les compositions destinées à l'alimentation animale, caractérisées en ce qu'elles renferment comme principe actif au moins l'un des composés définis à l'une quelconque des revendications 1 et 2.

10. Associations douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropanecarboxyliques, par les esters d'alcools 3-phénoxy benzyliques et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcools 3-phénoxy benzylique des acides 2-parachlorophényl 2-isopropyl acétiques, par les esters d'alléthrolones, d'alcool 3,4,5,6-tétrahydro phtalimido méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropanecarboxyliques, dans lesquels "halo" représente un atome de fluor, de chlore ou de brome, étant entendu que les composés de formule (I) peuvent exister sous toutes leurs formes stéréoisomères possibles de même que les copules acides et alcools des esters pyréthrinoïdes ci-dessus.

11. Procédé de préparation des composés de formule (I), tels que définis à l'une quelconque des revendications 1 et 2 caractérisé en ce que l'on soumet un acide de formule (II) : 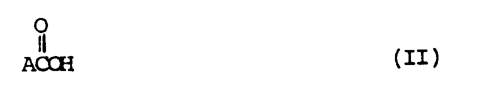 dans laquelle A est défini comme dans la revendication 1 ou un dérivé fonctionnel de cet acide à l'action d'un alcool de formule (III) : 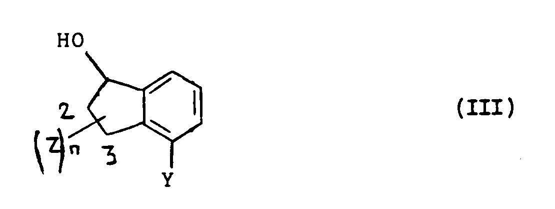 dans laquelle Y, Z et n sont définis comme précédemment pour obtenir le composé de formule (I) correspondant.

## Claims

1. In all the possible stereoisomer forms, as well as their mixtures, the compounds of formula (I): 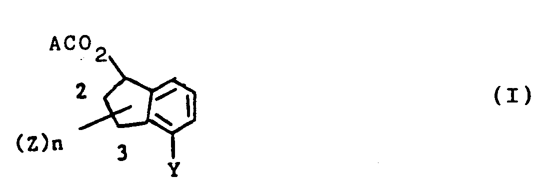 in which A represents the radical: 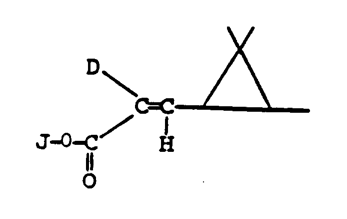 in which D represents a hydrogen or fluorine atom and J represents a saturated or unsaturated, linear, branched or cyclic alkyl radical containing up to 4 carbon atoms, optionally substituted by one or more halogen atoms,
- Z in position 2 or 3 represents a hydrogen atom, a fluorine atom, a saturated or unsaturated, linear, branched or cyclic alkyl radical containing up to 8 carbon atoms, n representing the number 1, 2 or 3;
- Y represents the radical -C≡N, CH₂CH=CH₂, CH₂-C≡CH or -CH₂-C≡N.

2. The compounds of formula (I) as defined in claim 1, of which the names follow:
- 4-(2-propenyl)-1-indanyl [1R-[1alpha(RS*),3alpha(E)]]-3-(3-ethoxy-2-fluoro-3-oxo-1-propenyl)-2,2-dimethyl cyclopropanecarboxylate.
- 4-(2-propenyl)-1-indanyl [1R-[1alpha(RS*),3alpha(Z)]]-3-[3-(1,1-dimethylethoxy)-3-oxo-1-propenyl-2,2-dimethyl cyclopropanecarboxylate.
- 4-(2-propenyl)-1-indanyl [1R-[1alpha,3alpha(E)]]-3-[3-(1,1-dimethylethoxy)-2-fluoro-3-oxo-1-propenyl]-2,2-dimethyl cyclopropanecarboxylate.
- 4-(2-propenyl)-1-indanyl [1R-[1alpha(R*),3alpha(E)]]-3-[3-(3-ethoxy-2-fluoro-3-oxo-1-propenyl)-2,2-dimethyl cyclopropanecarboxylate.
- 4-(2-propenyl)-1-indanyl [1R-[1alpha(R*),3alpha(E)]]-3-[3-(1,1-dimethylethoxy)-2-fluoro-3-oxo-1-propenyl]-2,2-dimethyl cyclopropanecarboxylate.
- 4-cyano-1-indanyl [1R-[1alpha(RS*),3alpha(E)]]-3-(3-ethoxy-2-fluoro-3-oxo-1-propenyl)-2,2-dimethyl cyclopropanecarboxylate.
- 4-cyano-1-indanyl [1R-[1alpha(RS*),3alpha(E)]]-3-[3-(1,1-dimethylethoxy)-2-fluoro-3-oxo-1-propenyl]-2,2-dimethyl cyclopropanecarboxylate.
- 4-(2-propynyl)-1-indanyl [1R-[1alpha(RS*),3alpha(E)]]-3-(ethoxy-2-fluoro-3-oxo-1-propenyl)-2,2-dimethyl cyclopropanecarboxylate.

3. The compounds of formula (I) as defined in any one of claims 1 and 2, for their use in combating parasites of vegetation, parasites of premises and parasites of warm-blooded animals.

4. Pesticide compositions intended for combating parasites of vegetation, parasites of premises and parasites of warm-blooded animals, characterized in that they contain as active ingredient at least one compound defined in any one of claims 1 and 2.

5. Insecticide compositions containing as active ingredient at least one of the compounds defined in any one of claims 1 and 2.

6. Acaricide compositions intended for combating parasitic acaridae of vegetation, containing as active ingredient at least one of the compounds defined in any one of claims 1 and 2.

7. Nematicide compositions containing as active ingredient at least one of the compounds defined in any one of claims 1 and 2.

8. Acaricide compositions intended for combating parasitic acaridae of animals, containing as active ingredient at least one of the compounds defined in any one of claims 1 and 2.

9. Compositions intended for animal fodder, characterized in that they contain as active ingredient at least one of the compounds defined in any one of claims 1 and 2.

10. Combinations endowed with insecticide, acaricide or nematicide activity, characterized in that they contain as active ingredient, on the one hand at least one of the compounds of general formula (I), and on the other hand at least one of the pyrethrinoid esters chosen from the group constituted by the esters of allethrolones, of 3,4,5,6-tetrahydrophthalimido methyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohols with 2,2-dimethyl-3-(2-oxo-3-tetrahydrothiophenylidene methyl)-cyclopropanecarboxylic acids, by the esters of 3-phenoxybenzyl alcohol and of alpha-cyano-3-phenoxybenzyl alcohols with 2,2-dimethyl-3-(2,2-dichlorovinyl)-cyclopropanecarboxylic acids, by the esters of alpha-cyano-3-phenoxy-benzyl alcohols with 2,2-dimethyl-3-(2,2-dibromovinyl)-cyclopropanecarboxylic acids, by the esters of 3-phenoxybenzyl alcohols with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with 2,2-dimethyl-3-(1,2,2,2-tetrahaloethyl)-cyclopropanecarboxylic acids, in which "halo" represents a fluorine, chlorine or bromine atom, it being understood that the compounds of formula (I) can exist in all their possible stereoisomer forms as well as the acid and alcohol copulas of the above pyrethrinoid esters.

11. Preparation process for compounds of formula (I), as defined in any one of claims 1 and 2, characterized in that an acid of formula (II): 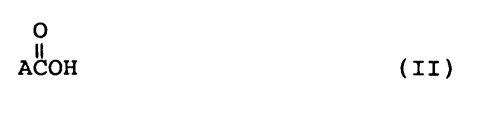 in which A is defined as in claim 1, or a functional derivative of this acid, is subjected to the action of an alcohol of formula (III): 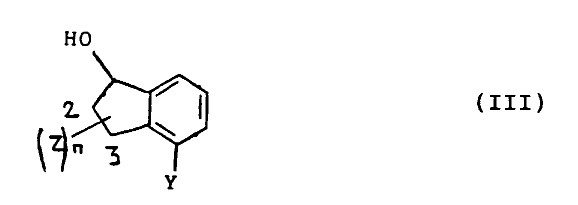 in which Y, Z and n are defined as previously, in order to obtain the corresponding compound of formula (I).

## Patentansprüche

1. In sämtlichen ihrer möglichen isomeren Formen sowie deren Gemischen die Verbindungen der Formel (I) 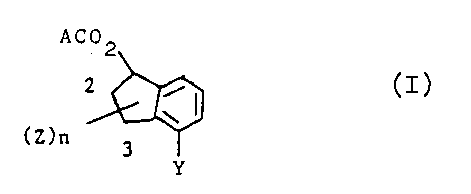 worin A den Rest 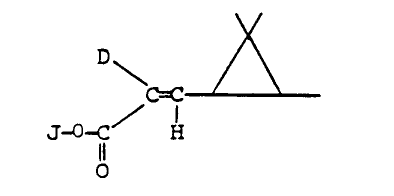 wiedergibt, worin D ein Wasserstoffatom oder Fluoratom bedeutet und J einen gesättigten oder ungesättigten, linearen, verzweigten oder cyclischen Alkylrest mit bis zu 4 Kohlenstoffatomen, der gegebenenfalls durch ein oder mehrere Halogenatome substituiert ist, bedeutet,
Z in 2- oder 3-Stellung ein Wasserstoffatom, ein Fluoratom, einen gesättigten oder ungesättigten, linearen, verzweigten oder cyclischen Alkylrest mit bis zu 8 Kohlenstoffatomen wiedergibt, wobei n die Zahl 1, 2 oder 3 bedeutet,
Y den Rest -C≡N, CH₂CH=CH₂, CH₂-C≡CH oder -CH₂-C≡N bedeutet.

2. Verbindungen der Formel (I), wie in Anspruch 1 definiert, mit den folgenden Bezeichnungen:
4-(2-Propenyl)-1-indanyl-[1R-[1α(RS*),3α(E)]]-3-(3-ethoxy-2-fluor-3-oxo-1-propenyl)-2,2-dimethylcyclopropancarboxylat,
4-(2-Propenyl)-1-indanyl-[1R-[1α(RS*),3α(Z)]]-3-[3-(1,1-dimethylethoxy)-3-oxo-1-propenyl-2,2-dimethylcyclopropancarboxylat,
4-(2-Propenyl)-1-indanyl-[1R-[1α,3α(E)]]-3-[3-(1,1-dimethylethoxy)-2-fluor-3-oxo-1-propenyl]-2,2-dimethylcyclopropancarboxylat,
4-(2-Propenyl)-1-indanyl-[1R-[1α(R*),3α(E)]]-3-(3-ethoxy-2-fluor-3-oxo-1-propenyl)-2,2-dimethylcyclopropancarboxylat,
4-(2-Propenyl)-1-indanyl-[1R-[1α(R*),3α(E)]]-3-[3-(1,1-dimethylethoxy)-2-fluor-3-oxo-1-propenyl]-2,2-dimethylcyclopropancarboxylat,
4-Cyano-1-indanyl-[1R-[1α(RS*),3α(E)]]-3-(3-ethoxy-2-fluor-3-oxo-1-propenyl)-2,2-dimethylcyclopropancarboxylat,
4-Cyano-1-indanyl-[1R-[1α(RS*),3α(E)]]-3-[3-(1,1-dimethylethoxy)-2-fluor-3-oxo-1-propenyl]-2,2-dimethylcyclopropancarboxylat,
4-(2-Propinyl)-1-indanyl-[1R-[1α(RS*),3α(E)]]-3-(ethoxy-2-fluor-3-oxo-1-propenyl)-2,2-dimethylcyclopropancarboxylat.

3. Verbindungen der Formel (I), wie in einem der Ansprüche 1 und 2 definiert, für deren Verwendung bei der Bekämpfung von Parasiten der Pflanzen, Parasiten von Räumlichkeiten und Parasiten warmblütiger Tiere.

4. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eine Verbindung, wie in einem der Ansprüche 1 und 2 definiert, enthalten.

5. Insektizide Zusammensetzungen, enthaltend als Wirkstoff zumindest eine der Verbindungen, wie in einem der Ansprüche 1 und 2 definiert.

6. Akarizide Zusammensetzungen für die Bekämpfung von Milbenparasiten der Pflanzen, enthaltend als Wirkstoff zumindest eine der Verbindungen, wie in einem der Ansprüche 1 und 2 definiert.

7. Nematizide Zusammensetzungen, enthaltend als Wirkstoff zumindest eine der Verbindungen, wie in einem der Ansprüche 1 und 2 definiert.

8. Akarizide Zusammensetzungen für die Bekämpfung von Milbenparasiten der Tiere, enthaltend als Wirkstoff zumindest eine der Verbindungen, wie in einem der Ansprüche 1 und 2 definiert.

9. Zusammensetzungen für die tierische Ernährung, dadurch gekennzeichnet, daß sie als Wirkstoff zumindest eine der Verbindungen, wie in einem der Ansprüche 1 und 2 definiert, enthalten.

10. Assoziationen mit insektizider, akarizider oder nematizider Aktivität, dadurch gekennzeichnet, daß sie als Wirkstoff einesteils zumindest eine der Verbindungen der allgemeinen Formel (I) und anderenteils zumindest einen der Pyrethrinoidester, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, den Estern der 5-Benzyl-3-furylmethylalkohole der 2,2-Dimethyl-3-(2-oxo-3-tetrahydrothiophenylidenmethyl)-cyclopropan - carbonsäuren, den Estern der 3-Phenoxybenzylalkohole und der α-Cyano-3-phenoxybenzylalkohole der 2,2-Dimethyl-3-(2,2-dichlorvinyl)-cyclopropan- carbonsäuren, den Estern der α-Cyano 3-phenoxybenzylalkohole der 2,2-Dimethyl-3-(2,2-dibromvinyl)-cyclopropan- carbonsäuren, den Estern der 3-Phenoxybenzylalkohole der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan - carbonsäuren, enthalten, worin "halo" ein Fluor-, Chlor- oder Bromatom wiedergibt, mit der Maßgabe, daß die Verbindungen (I)in sämtlichen ihrer möglichen stereoisomeren Formen ebenso wie die sauren Verknüpfungskomponenten und die Alkohole der vorstehenden Pyrethrinoidester vorliegen können.

11. Verfahren zur Herstellung der Verbindungen der Formel (I), Wie in einem der Ansprüche 1 und 2 definiert, dadurch gekennzeichnet, daß man eine Säure der Formel (II) 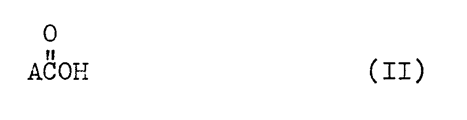 worin A wie in Anspruch 1 definiert ist, oder ein funktionelles Derivat dieser Säure der Einwirkung eines Alkohols der Formel (III) 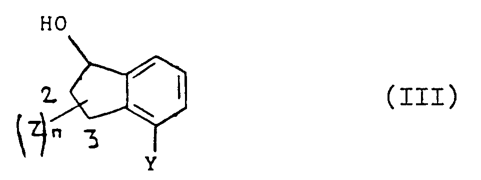 worin Y, Z und n wie vorstehend definiert sind, unterzieht, um zu der entsprechenden Verbindung der Formel (I) zu gelangen.
